(19) Europäisches Patentamt
European Patent Office
Office européen des brevets

(11) Publication number: **0 362 556 B1**

(12) # EUROPEAN PATENT SPECIFICATION

(45) Date of publication of patent specification: **02.11.94**

(51) Int. Cl.[5]: **C12P 41/00**, C12P 17/02, C07D 303/48

(21) Application number: **89116104.4**

(22) Date of filing: **31.08.89**

(54) **Method for preparing optically active 3-phenylglycidic acid esters.**

(30) Priority: **02.09.88 JP 221016/88**

(43) Date of publication of application:
**11.04.90 Bulletin 90/15**

(45) Publication of the grant of the patent:
**02.11.94 Bulletin 94/44**

(84) Designated Contracting States:
**AT BE CH DE ES FR GB GR IT LI LU NL SE**

(56) References cited:
**EP-A- 0 158 339**
**EP-A- 0 237 983**
**EP-A- 0 264 457**

**TETRAHEDRON, vol. 41, no. 7, 1985, pages 1393-1399, Pergamon Press Ltd., GB; P.MELLONI et al.**

(73) Proprietor: **TANABE SEIYAKU CO., LTD.**
**2-10, Dosho-machi 3-chome**
**Chuo-ku Osaka (JP)**

(72) Inventor: **Shibatani, Takeji**
**18-5, Uzumoridai 3-chome**
**Higashinada-ku**
**Kobe-shi Hyogo-ken (JP)**
Inventor: **Nakamichi, Katsuhiko**
**287-9, Hikishonishi-machi**
**Sakai-shi Osaka-fu (JP)**
Inventor: **Matsumae, Hiroaki**
**10-25, Morikita-machi, 7-chome**
**Higashinada-ku**
**Kobe-shi Hyogo-ken (JP)**

(74) Representative: **Hansen, Bernd, Dr.rer.nat. et al**
**Hoffmann, Eitle & Partner**
**Patentanwälte**
**Postfach 81 04 20**
**D-81904 München (DE)**

EP 0 362 556 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

This invention relates to a novel method for preparing optically active 3-phenylglycidic acid esters.

Optically active 3-phenylglycidic acid ester compounds are important ones as synthetic intermediates for dilthiazem hydrochloride useful as coronary vasodilator and other various pharmaceutical compounds, and in the prior art, as the method for preparing this compound, there has been known a method in which methyl trans-3-(4-methoxyphenyl)glycidate is hydrolyzed to a corresponding carboxylic acid, and the carboxylic acid is optically resolved with an optically active amine and then esterified (Japanese Unexamined Patent Publications Nos. 13775/1985 and 13776/1985).

However, the above method involves the drawback that many steps are included, and yet the desired optically active methyl trans-3-(4-methoxyphenyl)glycidate could be obtained only as an oil of low purity.

### SUMMARY OF THE INVENTION

The present inventors have studied intensively in order to solve such drawback, and consequently found a method capable of obtaining the desired optically active 3-phenylglycidic acid ester compounds at once and as crystals from racemic 3-phenylglycidic acid ester compounds, to accomplish the present invention.

More specifically, according to the present invention, a method is provided for preparing an optically active isomer of a trans-3-phenylglycidic acid ester compound of the formula:

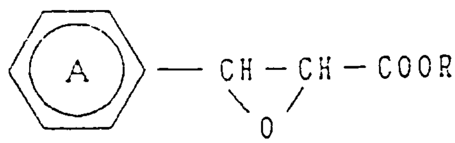

[ I ]

wherein Ring A is a substituted or unsubstituted phenyl group, and R is an ester residue, which comprises:

(a) permitting an enzyme derived from a microorganism belonging to the genus Corynebacterium or the genus Serratia, a culture broth of said microorganism, microbial cells collected from said culture broth or a processed product of said microbial cells, having the ability of stereoselectively hydrolyzing an ester bond to act on a racemic trans-3-phenylglycidic acid ester compound (I), thereby stereoselectively hydrolyzing one of optically active isomers thereof, and

(b) separating and collecting the antipode thereof from the reaction mixture.

### BRIEF DESCRIPTION OF THE DRAWINGS

Fig. 1 is an IR absorption spectrum of methyl (2R, 3S)-3-(4-methoyyphenyl)glycidate; Fig. 2 a NMR spectrum of the same compound; and Fig. 3 a mass spectrum of the same compound.

### DESCRIPTION OF THE PREFERRED EMBODIMENTS

Even when the 3-phenylglycidic acid ester compound represented by the formula [I] may have a substituent selected from a lower alkyl group, a lower alkoxy group and a halogen atom on the ring A, the method of the present invention can be practiced similarly to the case were no substituent exists on the ring A. Examples of such substituent may include methyl group, methoxy group or chloro atom at the 4-position. The ester residue R is ordinarily a lower alkyl group such as methyl, ethyl, isopropyl or t-butyl group.

In the present invention, as the racemic 3-phenylglycidic acid ester compound [I], not only one containing (2S, 3R) isomer and (2R, 3S) isomer in equal amounts, but any one which contains both of these optically active isomers can be used.

Examples of the enzyme to be used in the present invention include a group of hydrolases called lipase or esterase. These enzymes may be those extracted according to known methods from microorganism cells containing these hydrolases.

In the present invention, a culture broth of a microorganism producing the above-mentioned enzyme, microbial cells collected from said culture broth and a processed product of said microbial cells can also be

used as the enzyme source.

The microorganism used in the present invention belongs to the genus Corynebacterium or the genus Serratia and has the ability of producing the hydrolase as mentioned above. Specific examples of such microorganisms include, for example, *Corynebacterium alkanolyticum* ATCC 21511, *Corynebacterium hydrocarboclastum* ATCC 15592, *Corynebacterium primorioxydans* ATCC 31015, *Serratia liquefaciens* ATCC 27592, *Serratia marcescens* ATCC 13880, ATCC 14764, ATCC 19180, ATCC 21074, ATCC 27117 and ATCC 21212. These may be either wild species or mutant strains, and further may be those derived from these microorganisms according to the bioengineering methods such as gene recombination, cell fusion, etc.

Also, the culture broth and microbial cells of the above microorganism can be obtained by cultivating said microorganism in a medium conventionally used in this field of the art, for example, a medium containing carbon sources, nitrogen sources and inorganic salts conventionally used, at room temperature or under heating (preferably about 20 to 40 °C), and under aerobic conditions at pH of about 5 to 8 and, if necessary, separating and collecting the cells from the culture broth in conventional manner.

During cultivation, it is also possible to enhance the enzyme activity by adding the racemic 3-phenylglycidic acid ester compound (I) into the medium in an amount of about 0.001% or more, particularly about 0.1 to 1%.

As the processed product of such microbial cells, there may be included lyophilized cells, acetone dried cells, self-digested product of cells, cell extract, cell ground product, sonication treated product of cells of the above microorganism. Further, the microbial cells or processed product of cells of the present invention can be immobilized according to the known method such as the polyacrylamide method, the sulfur containing polysaccharide gel method (carrageenan gel method), the alginic acid gel method, the agar gel method, etc. before use.

The stereoselective hydrolysis reaction according to the present invention can be practiced by contacting the enzyme with a racemic 3-phenylglycidic acid ester compound [I] in an appropriate solvent.

The substrate concentration may be preferably 0.05 to 20%, above all 0.5 to 5%, and the reaction will suitably proceed at room temperature or under heating, preferably at 10 to 50 °C, particularly preferably 25 to 40 °C. During the reaction, it is preferable to adjust the pH of the reaction mixture to pH 5 to 10, paticularly 6 to 9. In this case, since most of the substrates are difficultly soluble in water, it is preferable to carry out the reaction in a two-phase solvent system of water or an aqueous solvent and an organic solvent. Examples of such organic solvent may include carbon tetrachloride, chloroform, dichloromethane, trichloroethylene, chlorobenzene, benzene, toluene, xylene, t-butyl methyl ether, diisopropyl ether, diethyl ether, methyl isobutyl ketone, methyl ethyl ketone, ethyl acetate, butyl acetate, n-propyl alcohol, isopropyl alcohol, n-butyl alcohol, t-butyl alcohol, etc., particularly preferably ethyl acetate, carbon tetrachloride and toluene. When the culture broth or the microbial cells are used as the enzyme source, it is preferred to carry out the above reaction in the presence of a surfactant in order to shorten the reaction time or increase yield of the optically active 3-phenylglycidic acid ester compound [I]. As such surfactant, cetylpyridinium bromide, cetyltrimethyl-ammonium bromide, polyethylene glycol, polyoxyethylene octylphenyl ether, etc. can be used, and its amount added may be preferably about 0.0001 to 0.1% based on the reaction mixture.

Isolation of the optically active 3-phenylglycidic acid ester compound [I] from the reaction mixture thus obtained can be easily practiced according to the conventional method. For example, when hydrolysis reaction is carried out in a water-organic solvent two-phase system, one optically active isomer of the 3-phenylglycidic acid ester compound [I] is hydrolyzed to be migrated into the aqueous layer, while the other optically active isomer not subjected to the reaction remains in the organic solvent, and therefore the optically active 3-phenylglycidic acid ester compound can be collected as crystals by separating the organic solvent layer and subjecting it to concentration under reduced pressure.

According to the present invention, the optically active 3-phenylglycidic acid ester compound [I] can be obtained in short steps and yet as crystals of high purity, and therefore the method can be a commercially advantageous preparation method.

Example 1

Fifty (50) ml of a medium (pH 7.0) containing 0.5% glucose, 1% peptone, 1% meat extract, 1.25% yeast extract and 0.5% sodium chloride were placed in a 500 ml volume shaking flask and sterilized at 120 °C for 10 minutes. Into the medium, one platinum loop of *Serratia marcescens* ATCC 27117 was inoculated, and cultivation was performed under shaking at 30 °C for 20 hours. The microbial cells collected by centrifugation from 5.4 liters of the above culture were suspended in physiological saline and further the microbial cells were collected by centrifuga-tion. The microbial cells were suspended in 1.8 liters of 10 mM

phosphate buffer (pH 7.5) containing 0.01% cetyltrimethylammonium bromide, and added into 1.8 liters of carbon tetrachloride containing 7.2 g of racemic methyl 3-(4-methoxyphenyl)glycidate. Then, the stereoselective hydrolysis reaction was carried out at 30 °C for 3 days, whereby methyl (2S, 3R)-3-(4-methoxyphenyl)-glycidate was completely hydrolyzed. The carbon tetra-choride layer was separated, then concentrated under reduced pressure to obtain 3.0 g of methyl (2R, 3S)-3-(4-methoxyphenyl)-glycidate as crude crystals. After 10 ml of isopropyl alcohol was added to 3.0 g of the crude crystals, the mixture was dissolved by heating at 80 °C under stirring for 20 minutes. After gradually cooled from 80 °C to 20 °C over 3 hours, the mixture was ice-cooled for one hour and the precipitated crystals were filtered to obtain 2.9 g of crystals of methyl (2R, 3S)-3-(4-methoxyphenyl)-glycidate.

M.P.: 87 - 88 °C

$[\alpha]_D^{20}$ : -207.08° (C = 1, methanol)

Purity: 100%

| Elemental analysis | Calcd.<br>Found | C: 63.45,<br>C: 63.44, | H: 5.81,<br>H: 5.80, | O: 30.74<br>O: 30.76 |
|---|---|---|---|---|

IR spectrum: Fig. 1

NMR spectrum: Fig. 2

Mass spectrum: Fig. 3

Example 2

Into the medium shown in Example 1 was inoculated each of the bacteria shown in Table 1, and cultured at 30 °C for 20 hours. The microbial cells collected from 45 ml of the above medium by centrifugation were suspended in physiological saline and then the cells were further collected by centrifugation. Said cells were suspended in 15 ml of 10 mM phosphate buffer (pH 7.5) containing 0.01% cetyltrimethylammonium bromide, and added into 15 ml of carbon tetrachloride containing 60 mg of racemic methyl 3-(4-methoxyphenyl)glycidate to carry out stereoselective hydrolysis at 30 °C for 3 days. After the reaction, the carbon tetrachloride layer was separated to obtain a reaction mixture containing methyl (2R, 3S)-3-(4-methoxy-phenyl)glycidate. The content of the (2R, 3S) isomer in the reaction mixture was as shown in Table 1, and substantially no (2S, 3R) isomer which is its antipode was detected in the reaction mixture.

Quantitation of the above optical isomer was conducted by high performance liquid chromatography by use of a chiral cell OJΦ 4.6 x 250 mm manufactured by Dicel Kagaku Kogyo K.K. (hereinafter the same).

Table 1

| Microorganism used | (2R, 3S) isomer content (mg/ml) |
|---|---|
| *Achromobacter cycloclastes* IAM 1013 | 0.8 |
| *Alcaligenes faecalis* OUT 8030 | 0.7 |
| *Bacillus sphaericus* IFO 3525 | 0.7 |
| *Bacillus subtilis* OUT 8104 | 0.8 |
| *Brevibacterium ketoglutamicum* ATCC 15588 | 0.7 |
| *Corynebacterium alkanolyticum* ATCC 21511 | 1.8 |
| *Corynebacterium primorioxidans* ATCC 31015 | 1.8 |
| *Providencia alcalifaciens* JCM 1673 | 1.8 |
| *Pseudomonas mutabilis* ATCC 31014 | 1.8 |
| *Pseumodonas putida* ATCC 17453 | 1.8 |
| *Serratia liquefaciens* ATCC 27592 | 1.6 |
| *Serratia marcescens* ATCC 27117 | 1.8 |

## Claims

**1.** A method for preparing an optically active isomer of a trans-3-phenylglycidic acid ester compound of the formula:

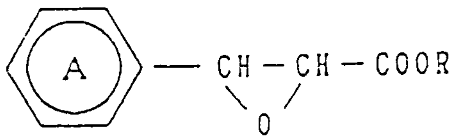

( I )

wherein Ring A is a substituted or unsubstituted phenyl group, and R is an ester residue, which comprises:

(a) permitting an enzyme derived from a microorganism belonging to the genus Corynebacterium or the genus Serratia, a culture broth of said microorganism, microbial cells collected from said culture broth or a processed product of said microbial cells, having the ability of stereoselectively hydrolyzing an ester bond to act on a racemic trans-3-phenylglycidic acid ester compound (I), thereby stereoselectively hydrolyzing one of optically active isomers thereof, and

(b) separating and collecting the antipode thereof from the reaction mixture.

**2.** A method according to Claim 1, wherein the enzyme is an esterase or a lipase.

**3.** A method according to claim 1, wherein the configuration of the antipode to be separated and collected is (2R, 3S).

**4.** A method according to claim 1, wherein the antipode to be separated and collected is a lower alkyl (2R, 3S)-3-(4-methoxyphenyl)glycidate.

**5.** A method according to claim 1, wherein the antipode to be separated and collected is methyl (2R, 3S)-3-(4-methoxyphenyl)glycidate.

**6.** A method according to Claim 1, wherein the stereoselective hydrolysis reaction is practiced by contacting the enzyme with the racemic trans-3-phenylglycidic acid ester compound in a solvent.

**7.** A method according to Claim 1, wherein the enzyme is derived from the culture broth of the microorganism, microbial cells collected from said culture broth or a processed product of said microbial cells.

**8.** A method according to Claim 1, wherein the microorganism is a microorganism belonging to the genus Serratia.

**9.** A method according to claim 6, wherein substrate concentration is 0.05 to 20%.

**10.** A method according to claim 6, wherein the reaction is practised in a two-phase solvent system of water or an aqueous solvent and an organic solvent.

**11.** A method according to claim 10, wherein the organic solvent is selected from the group consisting of carbon tetrachloride, chloroform, dichloromethane, trichloroethylene, chlorobenzene, benzene, toluene, xylene, t-butyl methyl ether, diisopropyl ether, diethyl ether, methyl isobutyl ketone, methyl ethyl ketone, ethyl acetate, butyl acetate, n-propyl alcohol, isopropyl alcohol, n-butyl alcohol and t-butyl alcohol.

## Patentansprüche

**1.** Verfahren zur Herstellung eines optisch aktiven Isomers einer Trans-3-Phenylglycidinsäureesterverbindung der Formel:

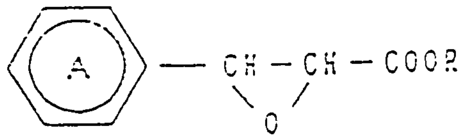

(I)

worin Ring A eine substituierte oder unsubstituierte Phenylgruppe ist und R ein Esterrest ist, welches umfaßt:

a) Einwirkenlassen eines Enzyms, das abgeleitet ist von einem Mikroorganismus der Gattung Corynebacterium oder der Gattung Serratia, einer Kulturbrühe dieses Mikroorganismus, mikrobiellen Zellen, die aus dieser Kulturbrühe gewonnen werden, oder eines Verarbeitungsprodukts dieser mikrobiellen Zellen, das die Fähigkeit besitzt, eine Esterbindung stereoselektiv zu hydrolysieren, auf eine racemische Trans-3-Phenylglycidinsäureesterverbindung (I), wodurch stereoselektiv eines ihrer optisch aktiven Isomere hydrolysiert wird, und

(b) Abtrennen und Gewinnen der Antipode davon aus der Reaktionsmischung.

**2.** Verfahren gemäß Anspruch 1, wobei das Enzym eine Esterase oder Lipase ist.

**3.** Verfahren gemäß Anspruch 1, wobei die Konfiguration der abgetrennten und gewonnenen Antipode die (2R,3S) - Konfiguration ist.

**4.** Verfahren gemäß Anspruch 1, wobei die abgetrennte und die gewonnene Antipode ein Niederalkyl-(2R,3S)-3-(4-methoxyphenyl)glycidat ist.

**5.** Verfahren gemäß Anspruch 1, wobei die abgetrennte und gewonnene Antipode Methyl-(2R,3S)-3-(4-methoxyphenyl)-glycidat ist.

**6.** Verfahren gemäß Anspruch 1, wobei die stereoselektive Hydrolysereaktion ausgeführt wird, indem das Enzym mit der racemischen Trans-3-Phenylglycidinsäureesterverbindung in einem Lösungsmittel in Kontakt gebracht wird.

**7.** Verfahren gemäß Anspruch 1, wobei das Enzym aus der Kulturbrühe des Mikroorganismus, von mikrobiellen Zellen, die aus dieser Kulturbrühe gewonnen wurden, oder aus einem Verarbeitungsprodukt dieser mikrobiellen Zellen abstammt.

**8.** Verfahren gemäß Anspruch 1, bei dem der Mikroorganismus ein Mikroorganismus der Gattung Serratia ist.

**9.** Verfahren gemäß Anspruch 6, bei dem die Substratkonzentration 0,05 bis 20 % beträgt.

**10.** Verfahren gemäß Anspruch 6, wobei die Reaktion in einem 2-phasigen Lösungsmittelsystem aus Wasser oder einem wäßrigen Lösungsmittel und einem organischen Lösungsmittel ausgeführt wird.

**11.** Verfahren gemäß Anspruch 10, bei dem das organische Lösungsmittel ausgewählt ist aus der Gruppe bestehend aus Tetrachlorkohlenstoff, Chloroform, Dichlormethan, Trichlorethylen, Chlorbenzol, Benzol, Toluol, Xylol, t-Butylmethylether, Diisopropylether, Diethylether, Methylisobutylketon, Methylethylketon, Ethylacetat, n-Butylacetat, n-Propylalkohol, Isopropylalkohol, n-Butylalkohol und t-Butylalkohol.

## Revendications

**1.** Procédé de préparation d'un isomère optiquement actif d'un ester d'acide trans-3-phénylglycidique de formule :

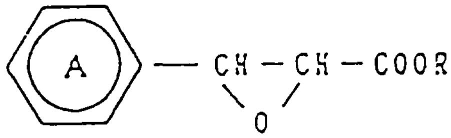

(I)

dans laquelle le cycle A est un groupe phényle substitué ou insubstitué, et R est un reste ester, qui consiste à :

(a) laisser une enzyme dérivée d'un micro-organisme appartenant au genre Corynebacterium ou au genre Serratia, d'un bouillon de culture desdits micro-organismes, de cellules microbiennes recueillies à partir dudit bouillon de culture ou d'un produit de traitement desdites cellules microbiennes, ayant la capacité d'hydrolyser stéréosélectivement une liaison ester, agir sur un ester d'acide trans-3-phénylglycidique racémique (I), pour hydrolyser ainsi stéréosélectivement un de ses isomères optiquement actif, et,

(b) séparer et recueillir son antipode du milieu réactionnel.

**2.** Procédé selon la revendication 1, dans lequel l'enzyme est une estérase ou une lipase.

**3.** Procédé selon la revendication 1, dans lequel la configuration de l'antipode à séparer et à recueillir est (2R, 3S).

**4.** Procédé selon la revendication 1, dans lequel l'antipode à séparer et à recueillir est un (2R, 3S)-3-(4-méthoxy-phényl)-glycidate d'alkyle inférieur.

**5.** Procédé selon la revendication 1, dans lequel l'antipode à séparer et à recueillir est le (2R, 3S)-3-(4-méthoxyphényl)-glycidate de méthyle.

**6.** Procédé selon la revendication 1, dans lequel la réaction d'hydrolyse stéréosélective est effectuée en mettant en contact l'enzyme et l'ester d'acide trans-3-phénylglycidique racémique dans un solvant.

**7.** Procédé selon la revendication 1, dans lequel l'enzyme est dérivée d'un bouillon de culture de micro-organisme, de cellules microbiennes recueillies dudit bouillon de culture ou d'un produit de traitement desdites cellules microbiennes.

**8.** Procédé selon la revendication 1, dans lequel le micro-organisme est un micro-organisme appartenant au genre Serratia.

**9.** Procédé selon la revendication 6, dans lequel la concentration du substrat est de 0,05 à 20%.

**10.** Procédé selon la revendication 6, dans lequel la réaction est effectuée dans un système de solvant à deux phases d'eau ou de solvant aqueux et de solvant organique.

**11.** Procédé selon la revendication 10, dans lequel le solvant organique est choisi dans le groupe constitué par le tétrachlorure de carbone, le chloroforme, le dichlorométhane, le trichloroéthylène, le chlorobenzène, le benzène, le toluène, le xylène, l'éther de t-butyle et de méthyle, l'éther di-isopropylique, l'éther diéthylique, la méthyl isobutyl cétone, la méthyl éthyl cétone, l'acétate d'éthyle, l'acétate de butyle, l'alcool n-propylique, l'alcool isopropylique, l'alcool n-butylique, et l'alcool t-butylique.

FIG. 1

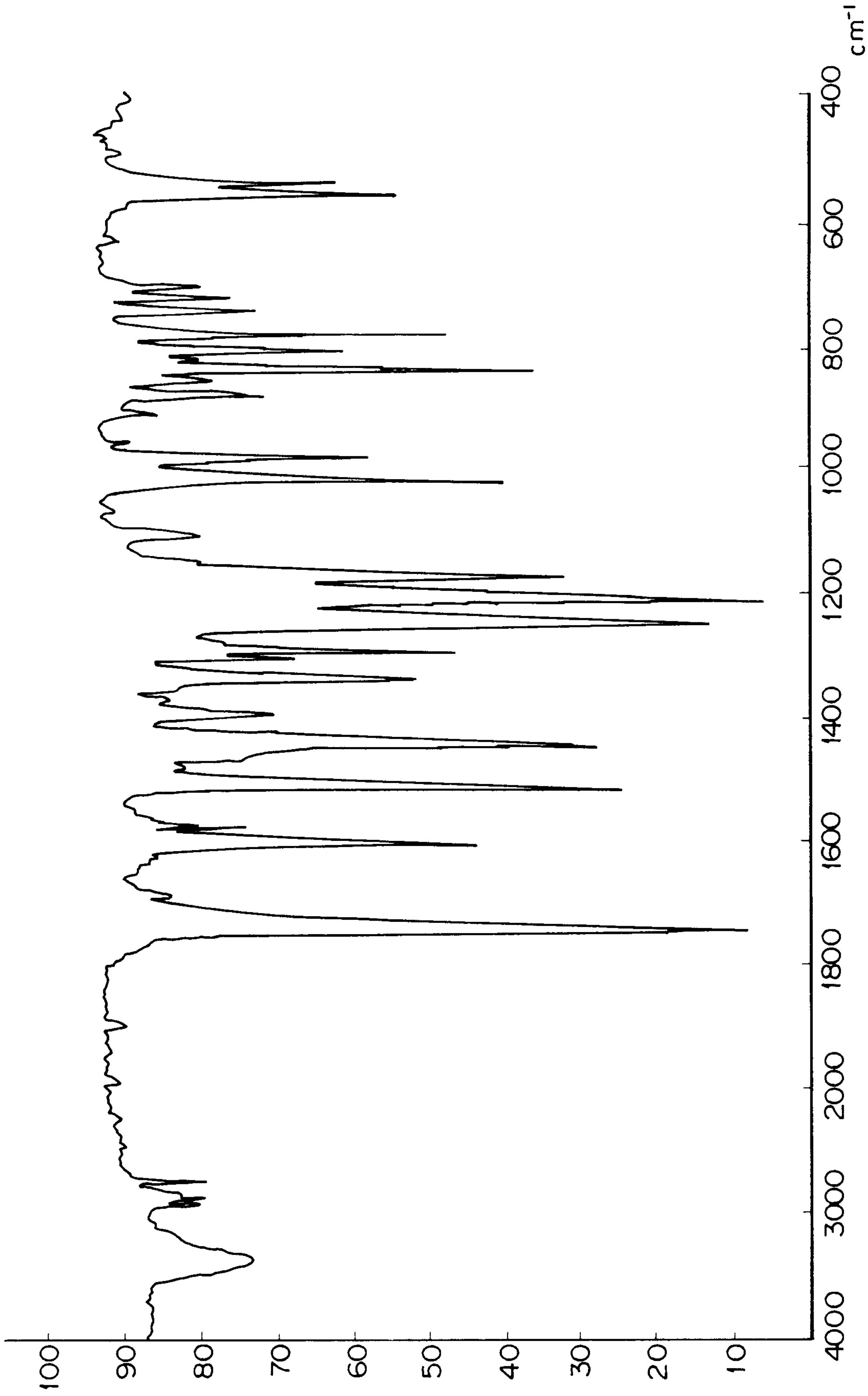
100
90
80
70
60
50
40
30
20
10
4000
3000
2000
1800
1600
1400
1200
1000
800
600
400
$cm^{-1}$

FIG. 2

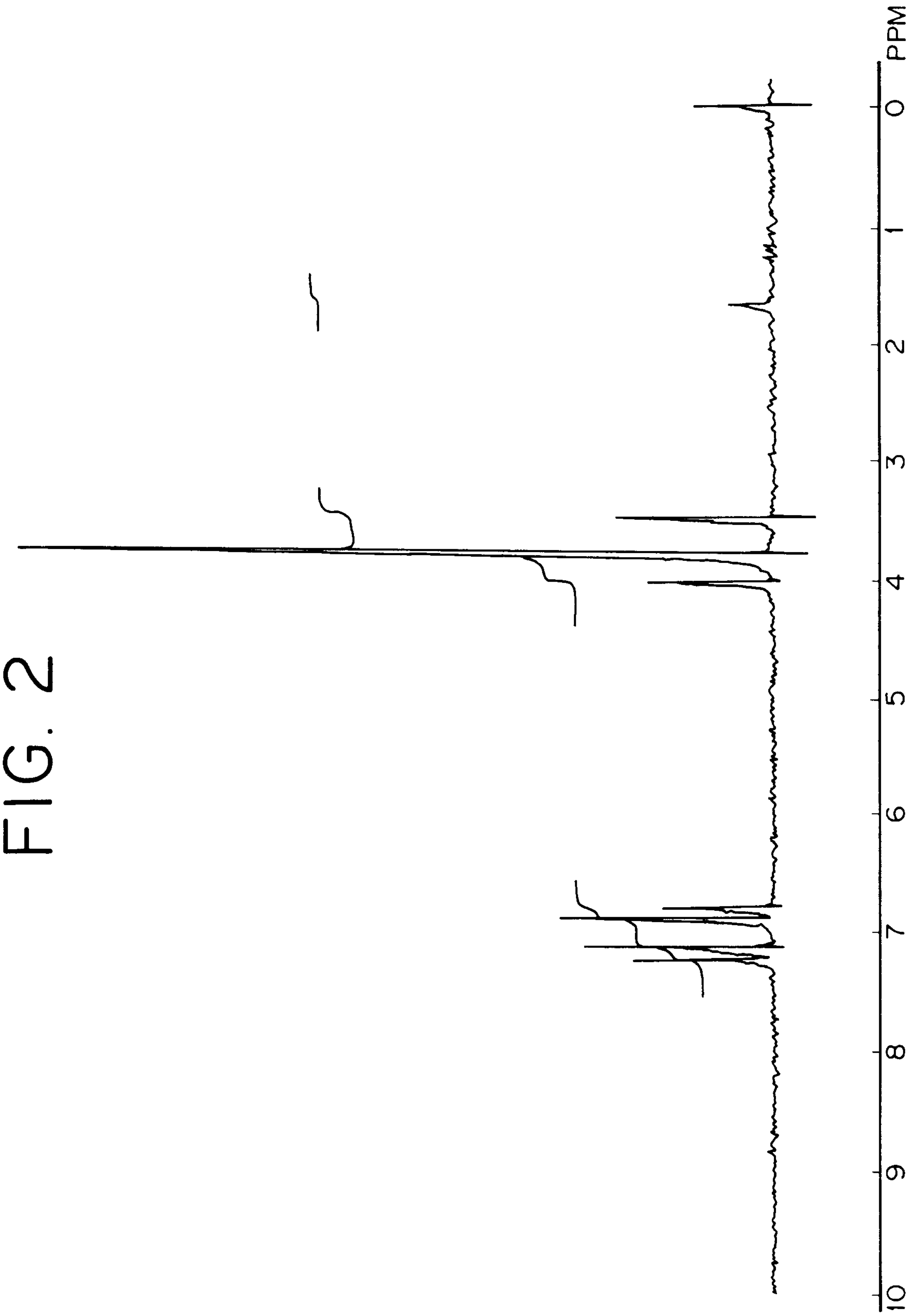

FIG. 3

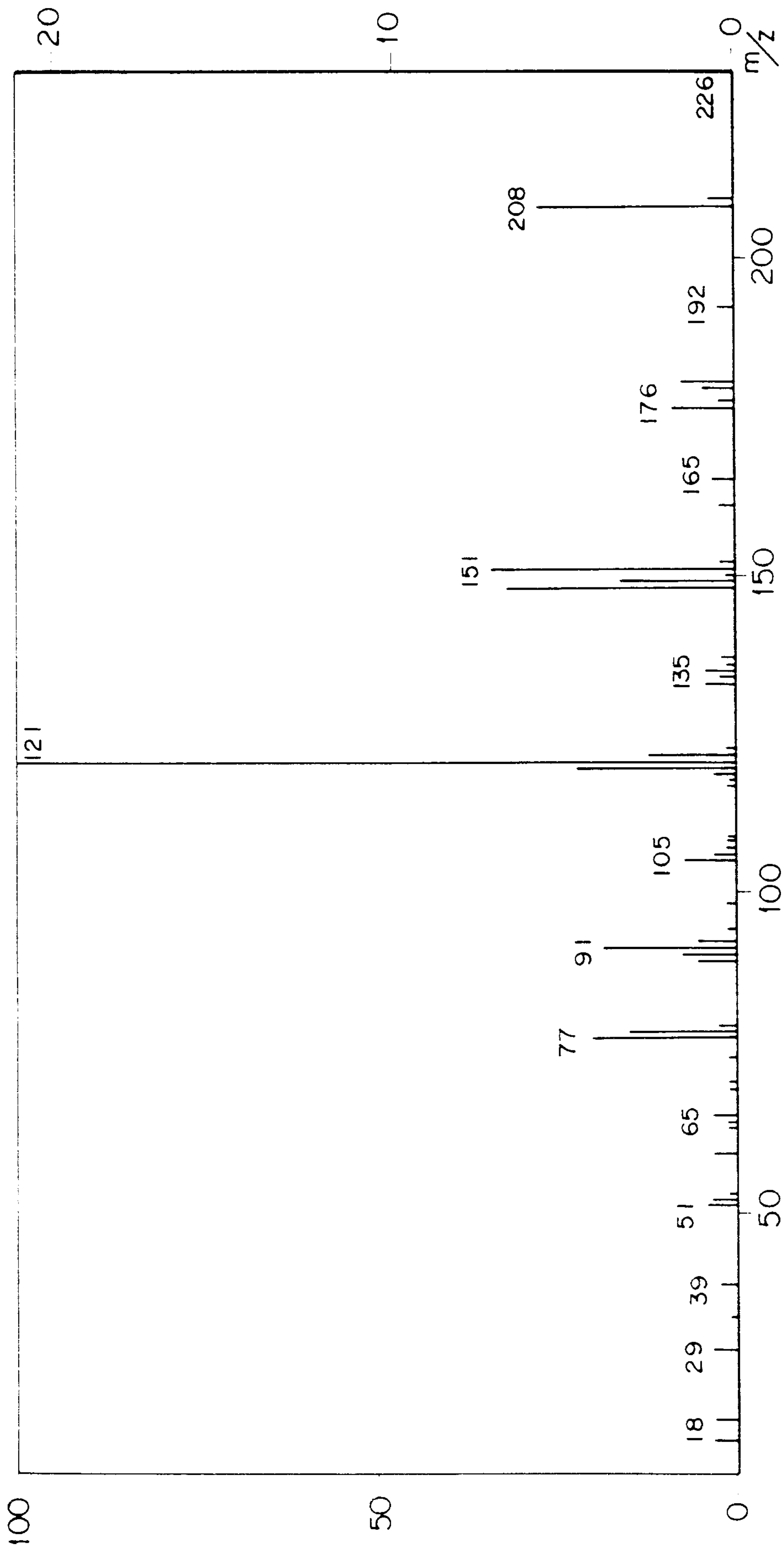

20
10
0
m/z
226
208
200
192
176
165
151
150
135
121
105
100
91
77
65
51
50
39
29
18
100
50
0